# EUROPEAN PATENT SPECIFICATION

(11) **EP 0 214 750 B1**
(45) Date of publication and mention of the grant of the patent: **22.09.1993**
(21) Application number: 86305901.0
(22) Date of filing: 31.07.1986
(51) Int. Cl.: C08G 59/40, C08L 63/00, C08G 73/10, C07D 403/04

(54) **Use of imides hardeners for epoxy resins and epoxy resin compositions containing these imides.**
Verwendung von Imiden als Härter für Epoxydharze und Epoxydharzzusammensetzungen, die diese Imide enthalten.
Utilisation des imides comme durcisseurs pour des résines époxydes et compositions de résines époxydes contenant ces imides.

(30) Priority: 31.07.1985 JP 170052/85; 12.03.1986 JP 53922/86
(43) Date of publication of application: 18.03.1987
(60) Divisional application: 93200242.1
(73) Proprietor: SUMITOMO CHEMICAL COMPANY, LIMITED, Chuo-ku Osaka 541 (JP)
(72) Inventor: Saito, Yasuhisa, Osaka-fu (JP); Kanagawa, Shuichi, Osaka (JP); Watanabe, Katsuya, Osaka-fu (JP); Kamio, Kunimasa, Osaka-fu (JP)
(74) Representative: Geering, Keith Edwin

(56) References cited:
- GB-A- 1 137 086
- US-A- 4 487 894
- Kirk-Othmer, Encyclopedia of Chemical Tecnology , 3rd Ed., vol 9, p.279, 1980, Wiley and Sons, New-York.

## Description

The present invention relates to the use of imide compounds as epoxy resin hardeners and to epoxy resin compositions which contain imide compound and epoxy resin and are suitable for lamination and molding.

Hitherto, for sealing laminates and semiconductor elements such as IC (integrated circuits) and LSI (large scale integrations) used in apparatus for industry and trade, epoxy resins have been used.

With laminates, however, there is a large change in dimension perpendicular to the substrate because of the low thermal resistance of the hardened epoxy resin, giving problems such as lowering in through-hole reliability, smear. With sealing material there is also the problem that when parts are connected to circuits by soldering, the laminates are cracked by the heat of the solder because of the large thermal expansion of the epoxy material.

Aromatic imide compounds have been used as hardener to improve the thermal resistance of such hardened epoxy product.

Generally, aromatic imide compounds are produced from aromatic tetracarboxylic acid anhydrides and aromatic diamines. The well-known representative aromatic tetracarboxylic acid anhydrides include pyromellitic acid anhydride, benzophenonetetracarboxylic acid anhydride. The aromatic imide compounds obtained from these acid anhydrides, however, have the problems that, generally, they are of very low solubility in low-boiling organic solvents, so that for dissolving them it is necessary to use special high-boiling solvents such as dimethylformamide, dimethylacetamide, N-methylpyrrolidone, dimethyl sulfoxide, cresol; and that they also have poor compatibility with epoxy resins, phenolic resins, amines, acid anhydrides, so that it is difficult to attain improvements in performances when using them with epoxy resins.

We have found that certain imide compounds having the structural unit 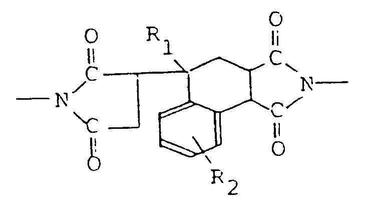
(wherein R₁ represents a hydrogen atom or a C₁-C₁₀ alkyl group, and R₂ represents a hydrogen atom, a C₁-C₂₀ alkyl or alkoxy group or a hydroxyl group) in the molecule are of good solubility and compatibility, and that the foregoing problems such as low thermal resistance, large changes in dimension, cracking of laminates by heat can be solved by using these imide compounds and epoxy resins together. GB-A-1137086 discloses some such compounds which are made by reacting TDA (3,4-dicarboxy-1,2,3,4-tetrahydro-1-naphthalene-succinic dianhydride) with (a) monoamine in 1:2 molar ratio or with (b) diamine in equimolar proportions.

The present invention provides the use of imide compound of formula (I), 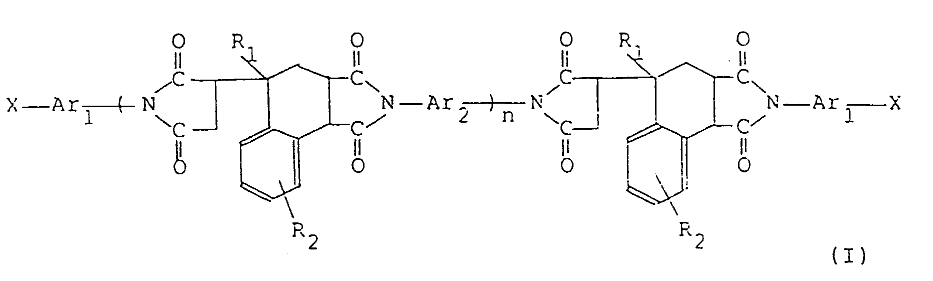
as an epoxy resin hardener,
wherein the X's are selected from hydroxyl and amino groups; the Ar₁'s, and the or each Ar₂ if present, are selected independently from aromatic residues; the or each R₁ is selected independently from hydrogen atom and C₁-C₁₀ alkyl groups; the or each R₂ is selected independently from a hydrogen atom, a hydroxyl group and C₁-C₂₀ alkyl and alkoxy groups; and n is zero or an integer of up to 30. The invention also provides an epoxy resin composition containing imide compound (B) and epoxy resin (A) as essential components.

Referring to Ar₁ and Ar₂ in more detail, they are independently mononuclear or polynuclear divalent aromatic residues of which the aromatic ring may or may not be substituted, e.g. with a lower alkyl group, a halogen atom, a lower alkoxy group, etc. Specifically, both Ar₁ and Ar₂ are the residues of aromatic amines. More specifically, the Ar₁'s are the residues of aromatic monoamines or diamines, and the Ar₂'s are the residues of aromatic diamines.

Of the aromatic amines, the aromatic diamines include for example: 4,4'-diaminodiphenylmethane, 3,3'-diaminodiphenylmethane, 4,4'diaminodiphenyl ether, 3,4'-diaminodiphenyl ether,4,4'-diaminodiphenylpropane,4,4'-diaminodiphenyl sulfone,3,3'-diaminodiphenyl sulfone,2,4-tolylenediamine,2,6-tolylenediamine,m-phenylenediamine, p-phenylenediamine, benzidine, 4,4'-diaminodiphenyl sulfide, 3,3'-dichloro-4,4'-diaminodiphenyl sulfone, 3,3'-dichloro-4,4'-diaminodiphenylpropane, 3,3'dimethyl-4,4'-diaminodiphenylmethane, 3,3'-dimethoxy-4,4'-diaminobiphenyl, 3,3'-dimethyl-4,4'-diaminobiphenyl, 1,3-bis(4-aminophemoxy)benzene, 1,3-bis(3-aminophenoxy)benzene, 1,4-bis(4-aminophenoxy)benzene, 2,2-bis(4-aminophenoxyphenyl)propane, 4,4'-bis(4-aminophenoxy)diphenyl sulfone, 4,4'-bis(3-aminophenoxy)diphenyl sulfone, 9,9'-bis(4-aminophenyl)anthracene, 9,9'-bis(4-aminophenyl)fluorene, 3,3'-dicarboxy-4,4'-diaminodiphenylmethane, 2,4-diaminoanisole, bis(3-aminophenyl)methylphosphine oxide, 3,3'-diaminobenzophenone, o-toluidine sulfone, 4,4'-methylene-bis-o-chloroaniline, tetrachlorodiaminodiphenylmethane, m-xylylenediamine, p-xylylenediamine, 4,4'-diaminostilbene, 5-amino-1-(4'-aminophenyl)-1,3,3-trimethylindane, 6-amino-1-(4'-aminophenyl)-1,3,3-trimethylindane, 5-amino-6-methyl-1-(3'-amino-4'-methylphenyl)-1,3,3-trimethylindane, 7-amino-6-methyl-1-(3'-amino-4'-methylphenyl)-1,3,3-trimethylindane, 6-amino-5-methyl-1-( 4'-amino-3'-methylphenyl)-1,3,3-trimethylindane, 6-amino-7-methyl-1-(4'-amino-3'-methylphenyl)-1,3,3-trimethylindane. These compounds may be used alone or in combination.

The aromatic monoamines include for example : o-aminophenol, m-aminophenol, p-aminophenol, 6-amino-m-cresol, 4-amino-m-cresol, 2,2-(4-hydroxyphenyl-4-aminophenyl)propane, 2,2-(4-hydroxyphenyl-2'-methyl-4'-aminophenyl)propane, 2,2-(3-methyl-4-hydroyyphenyl-4'-aminophenyl)propane, 8-amino-1-naphthol, 8-amino-2-naphtnol, 5-amino-1-naphthol, 4-amino-2-methyl-1-naphthol. These compounds may be used alone or in combination.

R₁ and R₂ are as defined above, but particularly preferably, R₁ is a C₁-C₁₀ alkyl group.

When substituent X in formula (I) is an amino group, imide compound (I) can be synthesized by reacting an excess of aromatic diamine with compound of formula (III), 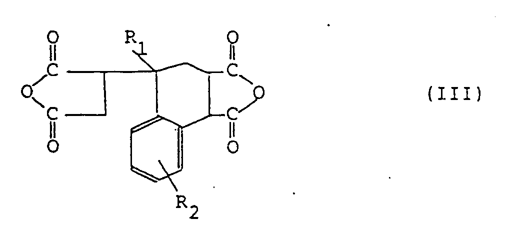
wherein R₁ and R₂ are as defined above, according to the common imidation technique.

When the substituent X in formula (I) is a hydroxyl group, imide compound (I) can be synthesized by adding aromatic monoamine having a hydroxyl group and aromatic diamine to compound (III) so that the molar ratio of aromatic diamine to compound (III) is n to (n+1) wherein n is as defined above and the molar ratio of aromatic monoamine to compound (III) is 2 to (n+1), and carrying out reaction according to the common imidation technique.

Compound (III) can be obtained by reacting compound of formula (IV) 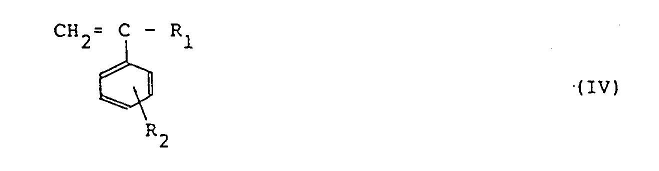
wherein R₁ and R₂ are as defined above, with maleic anhydride at a molar ratio of compound (IV) to maleic anhydride of 1 to 2 in the absence of radical polymerization catalyst and in the presence or absence off radical polymerization inhibitor. The compounds (IV) include styrene, α-methylstyrene, α,p-dimethylstyrene, α,m-dimethylstyrene, isopropylstyrene, vinyltoluene, p-tert-butylstyrene, p-isopropenylphenol, m-isopropenylphenol, 1-methoxy-3-isopropenylbenzene, 1-methoxy-4-isopropenylbenzene, vinylxylene. These compounds may be used alone or in combination.

The imide compounds (I) are soluble in high concentrations in low-boiling solvents such as acetone, methyl ethyl ketone, methyl isobutyl ketone, methyl and ethyl Cellosolve®, methylene chloride, chloroform. Also, they are of good compatibility with epoxy resins, phenolic resins, amines, acid anhydrides.

The imide compounds (I) are thermosettable by mixing with epoxy resins. The hardened product obtained has good thermal resistance, mechanical characteristics, solvent resistance.

The epoxy resin used in the present invention is for example a compound having two or more epoxy groups in the molecule. Examples of the epoxy resin include glycidyl ether compounds derived from dihydric or polyhydric phenols [e.g. bisphenol A, bisphenol F, hydroquinone, resorcinol, phloroglucinol, tris(4-hydroxyphenyl)methane, 1,1,2,2-tetrakis (4-hydroxyphenyl)ethane] or halogenated bisphenols (e.g. tetrabromobisphenol A); novolak type epoxy resins derived from novolak resins which are reaction products of phenols (e.g. phenol, o-cresol) with formaldehyde; amine type epoxy resins derived from aniline, p-aminophenol, m-aminophenol, 4-amino-m-cresol, 6-amino-m-cresol, 4,4'-diaminodiphenylmethane, 3,3'-diaminodiphenylmethane, 4,4'-diaminodiphenyl ether, 3,4'-diaminodiphenyl ether, 1,4-bis(4-aminophenoxy)benzene, 1,4-bis(3-aminophenoxy)benzene, 1,3-bis(4-aminophenoxy)benzene, 1,3-bis(3-aminophenoxy)benzene, 2,2-bis(4-aminophenoxyphenyl)propane, p-phenylenediamine, m-phenylenediamine, 2,4-tolylenediamine, 2,6-tolylenediamine, p-xylylenediamine, m-xylylenediamine, 1,4-cyclohexane-bis(methylamine), 1,3-cyclohexane-bis( methylamine), 5-amino-1-(4'aminophenyl)-1,3,3-trimethylindane, 6-amino-)-(4'-aminophenyl)-1,3,3-trimethylindane; glycidyl ester compounds derived from aromatic carboxylic acids (e.g. p-oxybenzoic acid, m-oxybenzoic acid, terephthalic acid, isophthalic acid); hydantoin type epoxy resins derived from 5,5-dimethylhydantoin; alicyclic epoxy resins such as 2,2'-bis(3,4-epoxycyclohexyl)propane, 2,2-bis[4-(2,3-epoxypropyl)cyclohexyl]propane, vinylcyclohexene dioxide, 3,4-epoxycyclohexylmethyl-3,4-epoxycyclohexanecarboxylate, etc.; and other compounds such as triglycidyl isocyanurate, 2,4,6-triglycidoxy-S-triazine. These epoxy resins may be used alone or in combination.

The epoxy resin composition of the present invention contain epoxy resin and imide compound (I) as essential components, and if necessary, may further contain well-known epoxy hardeners and hardening accelerators, fillers, flame retardants, reinforcing agents, surface-treating agents, pigments.

The well-known epoxy hardeners include for example amine type hardeners such as the foregoing aromatic amines (e.g. xylylene-diamine), aliphatic amines; polyphenols such as phenol novolak, cresol novolak; and acid anhydrides, dicyandiamide, hydrazides.

Preferably the total amount of (B) and other hardeners is 0.8 to 1.2 gram equivalents per gram equivalent of (A), and besides the amount of (B) is 0.4 to 1.2 gram equivalents per gram equivalent of (A). The hardening accelerators include for example amines [e.g. benzyldimethylamine, 2,4,6-tris(dimethylaminomethyl)phenol, 1,8-diazabicycloundecene], imidazole compounds (e.g. 2-ethyl-4-methylimidazole), boron trifluoride amine complexes. The fillers include silica, calcium carbonate; the flame retardants include aluminum hydroxide, antimony trioxide, red phosphorus; and the reinforcing agents include fabrics comprising organic or inorganic fibers (e.g. glass fibers, polyester fibers, polyamide fibers, alumina fibers), non-woven fabrics, mats, papers and combinations thereof.

The epoxy resin compositions of the invention can give hardened products of higher thermal resistance than obtainable by the prior art, and are of high value industrially as material for lamination and molding.

The present invention is illustrated in more detail by the following Examples, but it is not limited to these Examples.

### Preparative Example 1

To a flask equipped with a stirrer, a thermometer and a cooling separator were added 29.7 g (0.15 mole) of 4,4'-diaminodiphenylmethane and 242 g of m-cresol, and after dissolving the diaminodiphenylmethane in the m-cresol, 48.5 g of xylene was added. After heating the resulting mixture to a temperature of 120°C, 31.4 g (0.1 mole) of 1-methyl-3,4-dicarboxy-1,2,3,4-tetrahydro-1-naphthalenesuccinic acid anhydride was added at this temperature, and after raising the temperature to 175°C, dehydration was continued at this temperature for 5 hours. After completion of the reaction, a hexane/isopropanol mixed solution was added to precipitate the reaction product. The product was then washed two times with the same mixed solution and dried under reduced pressure to obtain an imide compound. This imide compound had a melting point of 241° C and an amine equivalent of 634 g/eq.

### Preparative Example 2

Example 1 was repeated except that 24.4 g (0.2 mole) of 2,4-tolylenediamine was used in place of 29.7 g (0.15 mole) of 4,4'-diaminodiphenylmethane, to obtain an imide compound. This compound had a melting point of about 220°C and an amine equivalent of 353 g/eq.

### Preparative Example 3

Example 1 was repeated - except that 24.0 g (0.22 mole) of m-aminophenol was used in place of 29.7 g (0.15 mole) of 4,4'-diaminodiphenylmethane, to obtain an imide compound. This compound had a melting point of about 210°C and a hydroxyl equivalent of 249 g/eq.

The imide compounds obtained in Examples 1 to 3 are soluble in solvents such as acetone, MEK, methylene chloride, methyl cellosolve. "Cellosolve" is a Registered Trade Mark.

### Example 4

100 Grams of Sumi® epoxy ELA-128 (bisphenol A type epoxy resin; epoxy equivalent, 187 g/eq; a product of Sumitomo Chemical Co.) and 163 g of the imide compound obtained in Example 1 were uniformly dissolved in 180 g of dimethylformamide. This solution was impregnated into glass cloth (WE 18K-BZ-2; a product of Nitto Boseki Co., Ltd.), which was then treated for 5 minutes in an oven kept at 180°C to obtain prepreg. Six pieces of prepreg and a copper foil (a product of Furukawa Circuit Foil Co.; TAI treatment, thickness, 35µ) were placed one upon another and press-molded at 180°C for 5 hours under a pressure of 50 kg/cm² to obtain a copper-lined laminate 1mm thick. The physical properties of this laminate were measured according to JIS C 6481 to obtain the result shown in Table 1.

### Example 5

Example 4 was repeated except that 90 g of the imide compound obtained in Example 2 was used in place of the imide compound obtained in Example 1, and that the amount of dimethylformamide gas changed from 180 g to 130 g, to obtain a laminate. The physical properties of this laminate are shown in Table 1.

### Comparative example 1

240 Grams of Sumi® epoxy ESA-011 (bisphenol A type epoxy resin; epoxy equivalent, 489 g/eq; a product of Sumitomo Chemical Co.), 20 g of Sumi® epoxy ESCN-220 (o-cresol novolak type epoxy resin; epoxy equivalent, 210 g/eq; a product of Sumitomo Chemical Co.), 9 g of dicyandiamide and 1 g of 2-phenyl-4-methyl-5-hydroxymethylimidazole were dissolved in a mixed solvent of 40 g of dimethylformamide, 60 g of ethylene glycol monomethyl ether and 60 g of methyl ethyl ketone. As in Example 4, the resulting solution was impregnated into glass cloth, which was then treated for 5 minutes in an oven kept at 160°C to prepare prepreg. This prepreg was press-molded under the same conditions as in Example 4 to prepare a laminate. The physical properties of the laminate are shown in Table 1.

**Table 1**

| Example | | Example 4 | Example 5 | Comparative example 1 |
|---|---|---|---|---|
| Tg | °C | 220 | 235 | 124 |
| Expansion coefficient in the Z direction (Tg or less) | 1/°C | 3.7× 10⁻⁵ | 3.6×10⁻⁵ | 5.7×10⁻⁵ |
| Thermal expansion rate in the Z direction (20° to 200°C) | % | 1.14 | 1.06 | 3.15 |
| " (20° to 260°C) | % | 2.41 | 2.33 | >10 |
| Water absorption (after 24 hours' boiling) | % | 1.18 | 1.16 | 1.94 |
| " (after 48 hours' boiling) | % | 1.29 | 1.24 | 2.18 |
| Resistance of copper foil to peeling | kg/m | 237 | 229 | 210 |
| Thermal resistance to soldering (300°C) | Appearance | Pass | Pass | Swelling |

### Example 6

100 Grams of Sumi ® epoxy ESCN-195XL (o-cresol novolak type epoxy resin, epoxy equivalent, 197 g/eq; a product of Sumitomo Chemical Co.), 62 g of the imide compound obtained in Example 2, 17 g of a phenol novolak resin, 1 g of 2,4,6-tris(dimethylaminomethyl)phenol, 1 g of carnauba wax, 2 g of a silane coupling agent (SH-6040; a product of Toray Silicone Ltd.) and 427 g of silica were kneaded at 100°C for 5 minutes on a two-roll kneader, cooled and pulverized to produce a molding material. This molding material was press-molded at 170°C for 5 minutes under a pressure of 70 kg/cm² and hardened for 5 hours in an oven kept at 180°C. The physical properties of the hardened product obtained are shown in Table 2.

### Comparative example 2

Example 6 was repeated except that 56 g at the phenol novolak resin only was used as a hardener, and that the amount at silica filler was changed from 427 g to 364 g, to obtain a hardened product. The physical properties of this hardened product are shown in Table 2.

**Table 2**

| Example | | Example 6 | Comparative example 2 |
|---|---|---|---|
| Tg | °C | 240 | 170 |
| Thermal expansion coefficient (<Tg) | 1/°C | 2.0×10⁻⁵ | 2.6×10⁻⁵ |
| Thermal expansion rate (20° to 260°C) | % | 1.10 | 1.35 |
| Bending strength ( 20°C) | kg/mm² | 14.7 | 14.8 |
| " (100°C) | " | 14.0 | 12.1 |
| " (150°C) | " | 12.4 | 9.0 |
| " (180°C) | " | 9.5 | 2.1 |

It is apparent from Tables 1 and 2 that the composition obtained according to the present invention has excellent thermal resistance, giving molded products having high dimensional stability.

## Claims

1. Use of imide compound of formula (I) 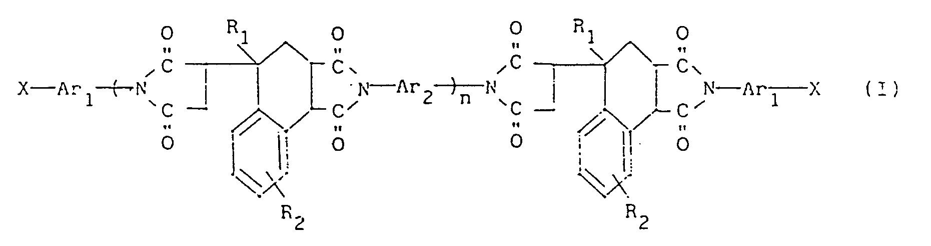 as an epoxy resin hardener,
wherein the X's are selected from hydroxyl and amino groups; the Ar₁'s, and the or each Ar₂ if present, are selected independently from aromatic residues; the or each R₁ is selected independently from a hydrogen atom and C₁-C₁₀ alkyl groups; the or each R₂ is selected independently from a hydrogen atom, a hydroxyl group, and C₁-C₂₀ alkyl and alkoxy groups; and n is zero or an integer of up to 30.

2. Use according to claim 1 wherein each X is amino.

3. Use according to claim 1 wherein each X is hydroxyl.

4. Use according to claim 1, 2 or 3 wherein R₁ is C₁-C₁₀ alkyl.

5. An epoxy resin composition containing epoxy resin and imide compound of formula (I) used according to any preceding claim.

6. The cured product of a composition according to claim 5.

## Patentansprüche

1. Verwendung von Imidverbindungen der Formel (I) 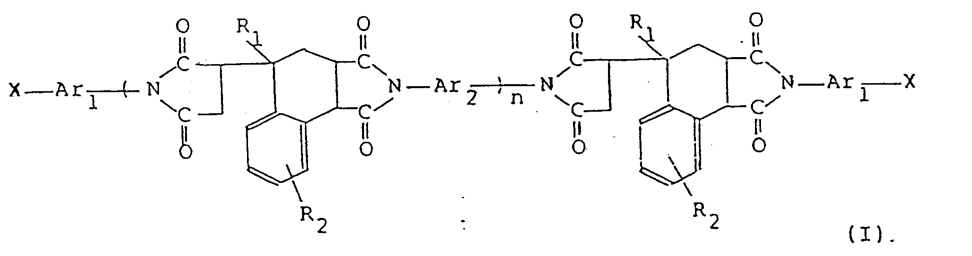 worin die Gruppen X aus Hydroxyl- und Aminogruppen gewählt sind, die Gruppen Ar₁ und die oder jede Gruppe Ar₂, falls vorhanden, unabhängig voneinander aus aromatischen Resten gewählt sind, der oder jeder Rest R₁ unabhängig voneinander aus Wasserstoffatomen und C₁-C₁₀-Alkylgruppen gewählt ist, der oder jeder Rest R₂ unabhängig voneinander aus einem Wasserstoffatom, einer Hydroxylgruppe und C₁-C₂₀-Alkyl- und Alkoxygruppen gewählt ist und n die Zahl Null oder eine ganze Zahl von bis zu 30 ist, als Härter für Epoxyharze.

2. Verwendung nach Anspruch 1, dadurch gekennzeichnet, daß jedes X Amino ist.

3. Verwendung nach Anspruch 1, dadurch gekennzeichnet, daß jedes X Hydroxyl ist.

4. Verwendung nach Anspruch 1, 2 oder 3, dadurch gekennzeichnet, daß R₁ C₁-C₁₀-Alkyl ist.

5. Epoxyharzzusammensetzung, enthaltend Epoxyharz und eine Imidverbindung der Formel (I), verwendet gemäß einem der vorhergehenden Ansprüche.

6. Gehärtetes Produkt einer Zusammensetzung nach Anspruch 5.

## Revendications

1. Utilisation d'un imide de formule (I) 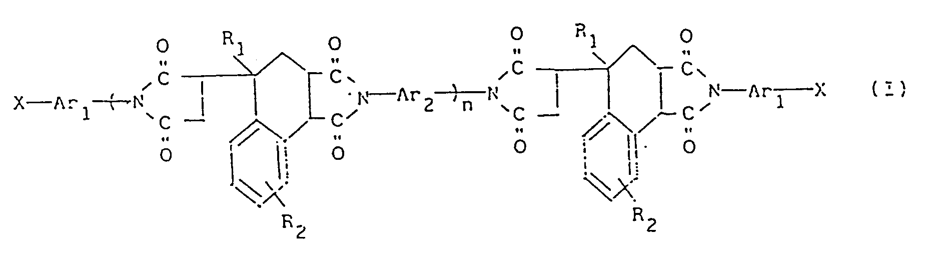 comme durcisseur pour résine époxy,
formule dans laquelle les groupes X sont choisis entre des groupes hydroxyle et amino ; les groupes Ar₁ et le groupe Ar₂ ou chaque groupe Ar₂ éventuellement présent sont choisis, indépendamment, entre des résidus aromatiques ; le groupe R₁ ou chaque groupe R₁ est choisi, indépendamment, entre un atome d'hydrogène et des groupes alkyle en C₁ à C₁₀ ; le groupe R₂ ou chaque groupe R₂ est choisi, indépendamment, entre un atome d'hydrogène, un groupe hydroxyle et des groupes alkyle et alkoxy en C₁ à C₂₀ ; et n est égal à zéro ou à un nombre entier allant jusqu'à 30.

2. Utilisation suivant la revendication 1, dans laquelle chaque X est un groupe amino.

3. Utilisation suivant la revendication 1, dans laquelle chaque X est un groupe hydroxyle.

4. Utilisation suivant la revendication 1, 2 ou 3, dans laquelle R₁ est un groupe alkyle en C₁ à C₁₀.

5. Composition de résine époxy, contenant une résine époxy et un imide de formule (I) utilise conformément à l'une quelconque des revendications précédentes.

6. Le produit de maturation d'une composition suivant la revendication 5.
